(19) Europäisches Patentamt
European Patent Office
Office européen des brevets

(11) **EP 3 897 173 B1**

(12) **EUROPEAN PATENT SPECIFICATION**

(45) Date of publication and mention
of the grant of the patent:
**10.12.2025 Bulletin 2025/50**

(21) Application number: **19829218.7**

(22) Date of filing: **20.12.2019**

(51) International Patent Classification (IPC):
*A23C 9/123* $^{(2006.01)}$    *A23C 9/13* $^{(2006.01)}$
*A23C 9/152* $^{(2006.01)}$    *A23C 11/10* $^{(2025.01)}$
*A23C 19/076* $^{(2006.01)}$    *C12R 1/46* $^{(2006.01)}$

(52) Cooperative Patent Classification (CPC):
(C-Sets available)
**A23C 9/1315; A23C 9/1234; A23C 9/1524;
A23C 19/0765; C12N 1/205;** A23C 2210/30;
C12R 2001/46                              (Cont.)

(86) International application number:
**PCT/EP2019/086880**

(87) International publication number:
**WO 2020/128084 (25.06.2020 Gazette 2020/26)**

(54) **WARM-FLAVOURED FERMENTED DAIRY PRODUCT COMPRISING EGG PROTEIN AND METHOD OF PREPARATION**

WARM-AROMATISIERTES FERMENTIERTES MILCHPRODUKT ENTHALTEND EI-PROTEIN UND VERFAHREN ZUR HERSTELLUNG

PRODUIT LAITIER FERMENTÉ À SAVEUR CHAUDE CONTENANT DES PROTÉINES D'OEUFS ET MÉTHODE DE PRÉPARATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.12.2018 EP 18215052**

(43) Date of publication of application:
**27.10.2021 Bulletin 2021/43**

(73) Proprietor: **Société des Produits Nestlé S.A.
1800 Vevey (CH)**

(72) Inventors:
• **RADIGUET, Sébastien
14100 Lisieux (FR)**
• **DUBOUX, Stéphane
1162 St-Prex (CH)**
• **ENAULT, Aurélie
14100 Lisieux (FR)**

(74) Representative: **Elleby, Gudrun
Société des Produits Nestlé S.A.
Avenue Nestlé 55
1800 Vevey (CH)**

(56) References cited:
**EP-A1- 3 178 329        EP-A2- 0 505 164
WO-A1-00/19831          WO-A1-2012/136833
CN-A- 102 952 758       CN-B- 102 986 872
US-A1- 2004 213 885     US-A1- 2009 304 863**

• **DATABASE FSTA [online] INTERNATIONAL
FOOD INFORMATION SERVICE (IFIS),
FRANkFURT-MAIN, DE; YOUNG-TAE KO: "The
effects of egg white powder addition on acid
production by lactic acid bacteria and quality of
curd yogurt.", XP002792519, Database
accession no. FS-1997-01-P-0105**
• **KOREAN JOURNAL OF FOOD SCIENCE AND
TECHNOLOGY, vol. 27, no. 4, 1995, DEP. OF
FOODS & NUTR., DUKSUNG WOMEN'S UNIV.,
SSANGMUN-DONG, DOBONG-KU, SEOUL
132-714, KOREA, pages 458 - 463**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
A23V 2002/00, A23V 2250/5428, A23V 2400/139,
A23V 2400/157

## Description

## TECHNICAL FIELD

The present invention generally relates to a process for preparing a fermented dairy product matching with warm flavouring agent, as well as to a fermented dairy product obtainable by such a process and a multilayer food product comprising said fermented dairy product. The present invention also relates to a mesophilic strain suitable for preparing said fermented dairy product.

## BACKGROUND OF THE INVENTION

Fermented dairy products, such as yoghurts, are available on the market with multiple textures and flavours. Consumers have a wide choice in the supermarket shelves among drinkable yoghurts, stirred yoghurts, set yoghurts, Greek-style yoghurts, whipped fermented dairy products and so on. Fermented dairy products may be plain, sweet, spicy, fruit-flavoured, or even vegetable-flavoured. In addition, some of them incorporate cereal preparations or exhibit a fruit preparation layer at the bottom or the top of the container.

The fermented dairy category is a highly competitive food category. This is why innovation and renovation are important to maintain or increase market share and to differentiate from the competitors.

Fermented dairy products flavoured with warm flavouring agents, such as chocolate or caramel, are scarcely represented on the market. The few fermented products with warm flavouring agents available on the market are not appreciated by a majority of consumers. Indeed, such combination is challenging mainly because of the inadequacy between warm flavouring agents and acidity coming from lactic fermentation of the dairy base. The tartness of fermented dairy products alters warm flavouring agents by providing an unpleasant aftertaste.

There is a need to provide fermented dairy products having a balanced sensory profile when admixed with a mixture exhibiting warm notes such as a chocolate sauce.

Limiting the acidity of fermented dairy products as perceived in mouth, appears key to obtain products where warm notes are in harmony with fermented notes. Examples of approaches for reducing the acidity perceived in mouth of fermented dairy products are disclosed in the prior art.

WO 98/12931 (CALPIS. CO. LTD) relates to a fermented milk with a starter culture of lactic acid bacteria containing *Lactobacillus helveticus* and *Lactobacillus acidophilus.* The co-fermenting system using *Lactobacillus acidophilus* enables to suppress the increase in acidity in the final product due to the enzymatic activity of *Lactobacillus helveticus* during low temperature storage. The increase in acidity is due to a continuous fermentation of remaining carbohydrates by *Lactobacillus helveticus* during storage. This phenomenon is amplified when the cold chain is not efficiently maintained.

The invention disclosed in WO 98/12931 requires the combination of two specific strains. This is constraining, not cost-efficient and hard to handle in an industrial setting. Moreover, the fermented milk product is not mixed with a warm flavouring agents. Therefore, it could not be concluded whether the resulting fermented milk product matches with warm flavouring agents.

WO 00/19831 (COMPAGNIE GERVAIS DANONE) relates to a fermented dairy product flavoured with a warm flavour having a Dornic acidity ranging from 20 to 80 °D and a pH between 4.5 and 5.5. The dairy raw material is treated in order to limit its buffering power by reducing its mineral salts content and/or its protein content. The resulting fermented dairy product matches with warm flavors without impairing them.

WO 2004/068958 A1 (COMPAGNIE GERVAIS DANONE) relates to a yoghurt with a two-phase structure incorporating a chocolate preparation. More especially, it comprises a first phase consisting of globules of free fat and a second phase consisting of fat globules connected to a mixed system of protein material and fatty material. The two-phase structure is obtained by admixing a homogenized cream with a yoghurt. Said yoghurt with a two-phase structure matches with warm flavors such as chocolate. According to this application, the free fat globules may have an effect of masking the acidity perceived in mouth of the yoghurt by lining the mouth.

US 2006/0068075 A1 (GENERAL MILLS) relates to a fermented dairy product composed of a fermented dairy base having a pH between 4.7 and 5.3, containing active cultures and a low water activity sweet brown base component (e.g chocolate) admixed within the fermented dairy base. The sweet brown base component has a water activity inferior to 0.85. The active cultures are screened in such a way that their enzymatic activity results in less than 0.2pH unit drop during refrigerated shelf-life.

WO 2012/136833 A1 (CHR HANSEN) relates to a composition suitable for preparing a dairy product comprising at least one mesophilic starter culture, such as a *Lactococcus* starter culture, and a *Lactobacillus rhamnosus* strain capable of imparting onto the dairy product an enhanced creamy flavor without affecting the rheology negatively, the fermentation time or the post-acidification of the dairy product.

WO 2002/07541 A1 (NESTLE) relates to an egg and-milk product having gelling and emulsifying properties and suitable for fermented foods of the drinks, dessert, fromage frais and set or creamy yoghurt type.

CN 102952758 A (BEIJING DQY AGRICULTURAL TECHNOLOGY) relates to a method for preparing an egg-milk fermented beverage by using a *Lactococcus lactis subsp. cremoris* strain (V401-403). The egg-milk fermented beverage is prepared by mixing liquid egg, pasteurized milk and a sweetening agent. The mixture is then filtered, such that an egg-milk mixed liquid is obtained. The egg-milk mixed liquid is then fermented after inoculating the *Lactoccocus lactis subsp. cremoris V401-403,* to obtain the egg-milk fermented beverage.

The post-acidifying properties of the *Lactoccocus lactis subsp. cremoris V401-403* strain are not reported in this document. In addition, this document only discusses a liquid beverage. It does not disclose an egg-and-milk powder.

None of the foregoing documents disclose the use of an egg-and-milk powder to limit the acidity perceived in mouth of fermented dairy products and therefore, to achieve a fermented dairy product matching with a warm flavouring agent such as chocolate.

Any reference to prior art documents in this specification is not to be considered an admission that such prior art is widely known or forms part of the common general knowledge in the field.

## SUMMARY OF THE INVENTION

The object of the present invention is to improve the state of the art, and in particular to provide a composition and a process that overcome the problems of the prior art and addresses the needs described above, or at least to provide a useful alternative.

The inventors were surprised to see that the object of the present invention could be achieved by the subject matter of the independent claims. The dependent claims further develop the idea of the present invention.

Accordingly, a first aspect of the invention proposes a process for the preparation of a fermented dairy product having at least one warm flavouring agent, a pH between 4.5 and 5.5 and a protein content between 2% and 4% based on the total weight of the fermented dairy product comprising the steps of:

a) preparing a dairy composition by mixing an egg-and-milk powder with a dairy base

b) homogenizing the dairy composition by high-pressure homogenization at a pressure ranging from 250 bar to 800 bar and a temperature ranging from 60°C to 70°C to obtain an homogenized dairy composition, pasteurizing the homogenized dairy composition at a temperature ranging from 85°C to 95°C for a time ranging from 3 minutes to 8 minutes to obtain a pasteurized dairy composition,

c) cooling the pasteurized dairy composition to a temperature between 32°C and 35°C, and then inoculating the pasteurized dairy composition with a mesophilic strain to obtain an inoculated dairy composition, wherein the mesophilic strain is a mesophilic strain having low post-acidifying properties, and wherein the mesophilic strain having low post-acidifying properties is from the genus Lactococcus and is selected from the group consisting of Lactococcus lactis subsp. lactis diacetylactis NCC 2216 having the accession number CNCM 1-5134, Lactococcus lactis subsp. lactis diacetylactis NCC 2272 having the accession number CNCM 1-5258, Lactococcus lactis subsp. Lactis NCC 2471 having the accession number NCIMB 8586 and Lactococcus lactis subsp. Lactis NCC 2205 having the accession number CNCM 1-5257,

d) fermenting the inoculated dairy composition at a temperature between 32°C and 35°C until a pH ranging from 4.5 to 5.5 is reached, to obtain a fermented dairy product,

e) mixing at least one warm flavouring agent during step a) and/or after step d), wherein the warm flavouring agent is selected among the group consisting of chocolate flavouring agent, vanilla flavouring agent, coffee flavouring agent, biscuit flavouring agent, cinnamon flavouring agent, caramel flavouring agent, praline flavouring agent, nougat flavouring agent, peanut flavouring agent, hazelnut flavouring agent, pistachio flavouring agent, almond flavouring agent, walnut flavouring agent, cashew nut flavouring agent and mixtures thereof,

f) optionally, cooling the fermented dairy product containing said warm flavouring agent to a temperature of 8°C.

In a second aspect, the invention proposes a fermented dairy product having a pH between 4.5 and 5.5 and a protein content ranging from 2% to 4% based on the total weight of the fermented dairy product comprising a mesophilic strain and at least one warm flavouring agent, obtainable by the process according to the first aspect of the invention, wherein the mesophilic strain is a mesophilic strain having low post-acidifying properties .

A third aspect of the invention proposes a multilayer food product comprising at least one layer of fermented dairy product according to the second aspect of the invention, or obtainable or obtained by the process according to the first aspect of the invention.

These and other aspects, features and advantages of the invention will become more apparent to those skilled in the art from the detailed description of embodiments of the invention, in connection with the attached drawing.

## BRIEF DESCRIPTION OF THE DRAWINGS

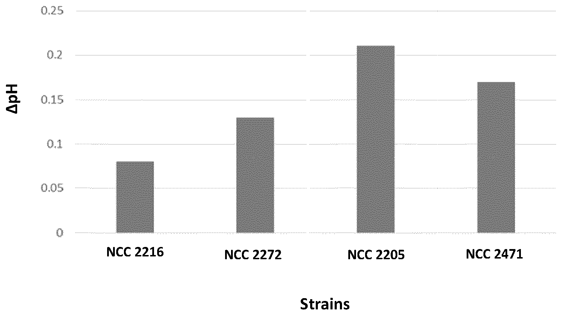

Figure 1 shows the assessment of the post-acidifying properties of four mesophilic strains (NCC 2216, NCC 2272, NCC 2205 and NCC 2471) in plain milk. The post-acidifying properties were assessed by determining the pH drop: delta pH.

## DETAILED DESCRIPTION OF THE INVENTION

As used in the specification, the words "comprise", "comprising" and the like are to be construed in an inclusive sense, that is to say, in the sense of "including, but not limited to", and do not exclude additional, unrecited elements or method steps. As used in the specification, the words "consisting of" and the like are to be construed in an exclusive or exhaustive sense: they exclude any unrecited element, step, or ingredient. As used in the specification, the words "consists essentially of" mean that specific further components can be present, namely those not materially affecting the essential characteristics of the invention.

As used in the specification, the term "substantially free" means that no more than about 10 weight percent, preferably no more than about 5 weight percent, and more preferably no more than about 1 weight percent of the excluded material is present. In a preferred embodiment, "substantially free" means that no more than about 0.1 weight percent of the excluded material remains. "Entirely free" typically means that at most only trace amount of the excluded material is present, and preferably, no detectable amount is present.

As used in the specification, the word "about" should be understood to apply to each bound in a range of numerals. Moreover, all numerical ranges should be understood to include each whole integer within the range.

As used in the specification, the singular forms "a", "an", and "the" include plural referents unless the context clearly dictates otherwise.

Unless noted otherwise, all percentages in the specification refer to weight percent, where applicable.

Unless defined otherwise, all technical and scientific terms have and should be given the same meaning as commonly understood by one of ordinary skill in the art to which this invention belongs.

In the context of the invention, the term "plant milk" refers to the liquid obtained after soaking, grinding and filtration of a plant material (e.g. almond, nut, rice, oat) or to an aqueous solution enriched with a plant protein concentrate or isolate (e.g. pea, soy). The term "aqueous solution" refers to a solution containing at least 90 wt% of water (e.g water, fruit or vegetable juice). The term "plant milk" also refers to the liquid obtained from high-starch legumes using the process disclosed in the patent application WO 2016/172570.

In the context of the invention , the term "high-starch legumes" refers to a legume which has greater than 10 wt% starch as measured on a dry weight basis (e.g. excluding the weight of water added to hydrate and process the legumes). Examples of high starch legumes include adzuki bean, chickpea, fava bean, and lentil.

In the context of the invention, the "overrun" is an indication of the volume of gas incorporated into a product. The overrun is defined as:

$$OR = \frac{Vm - Vo}{Vo} * 100$$

where Vo is the initial volume of a mass M of product, and Vm is the volume of the same mass M of product after incorporation of gas, for instance by whipping.

In the context of the invention, the acidity perceived in mouth relates to a sensory attributes measuring the intensity of the acid flavour. The acidity perceived in mouth is assessed by trained persons (e.g a trained panel) using a ranking from 1 (i.e. not acid, neutral) to 10 (very acid).

In a first aspect, the invention relates to a process for the preparation of a fermented dairy product having at least one warm flavouring agent, a pH between 4.5 and 5.5 and a protein content between 2% and 4% based on the total weight of the fermented dairy product. The fermented dairy product may be a drinkable fermented milk, a quark, a drinkable yoghurt or a spoonable yoghurt. Preferably, the fermented dairy product is a spoonable yoghurt. When the fermented dairy product is a spoonable yoghurt, it may be a set-style yoghurt, a stirred yoghurt or a Greek-style yoghurt.

In a first step, a dairy composition is prepared by mixing an egg-and-milk powder with a dairy base.

The dairy base may be a non-human mammal milk such as cow milk, sheep milk, buffalo milk, goat milk, donkey milk or camel milk. More especially, the non-human mammal milk may be whole milk, partially skimmed milk, fully skimmed milk, any powdered milk, any concentrated milk, any milk enriched with cream or any mixture thereof. The dairy base may alternatively be a plant milk such as almond milk, buckwheat milk, chestnut milk, coconut milk, hazelnut milk, hemp milk, legume milk, oat milk, peanut milk, quinoa milk, rice milk, soy milk, spelt milk and walnut milk. The dairy base may also be mixtures of at least one non-human mammal milk and at least one plant milk.

The dairy base may optionally comprise a dairy ingredient. In the context of the invention, the term "dairy ingredient" excludes the plant milks and the non-human mammal milks as previously disclosed in the specification. Examples of dairy ingredient include unfermented buttermilk, partially or totally dehydrated liquid buttermilk, concentrated whey, whey powder, whey proteins, concentrated whey proteins, water-soluble dairy proteins, dietary casein and caseinates manufactured from pasteurized products.

Food grade texturizing ingredients may also be added in the dairy base. The content of food grade texturizing ingredients used may vary depending upon the nature of the ingredient, the desired effect and the desired final texture. Examples of food grade texturizing ingredients suitable for the invention include gelatine, agar, guar gum, locust bean gum, xanthan, alginate, starch and mixtures thereof. Preferably, the fermented dairy product does not contain any food grade texturizing ingredients while remaining stable and keeping its organoleptic properties over the shelf life. The texturizing ingredients may be avoided to simplify the recipe of the fermented dairy product by reducing the number of ingredient and/or to simplify the process by removing the dosing step of the texturizing ingredients. Moreover, texturizing ingredients may also be avoided because consumers may have a bad image of such texturizing ingredients.

The dairy base may optionally comprise sweetening ingredients. The term "sweetening ingredients" excludes lactose naturally present in the milk of the dairy base. The fermented dairy product preferably contains a natural sweetening ingredient such as sucrose. Examples of natural sweetening ingredient include saccharose, lactose, honey, all types of glucose syrups or fructose syrups or natural extracts like stevia or rebaudioside A, and their combinations. The amount of sweetening ingredients used may vary depending upon the nature of the sweetener, the desired level of sweetness and the strength of the sweetener.

The dairy base may further be enriched with fruits, cereals, vitamins, minerals, fibers, prebiotics, probiotics and mixtures thereof.

The method for preparing the egg-and-milk powder will be described later in the specification herein-under.

In a second step, the dairy composition is homogenized by high-pressure homogenization to obtain a homogenized dairy composition. The pressure applied to the dairy composition ranges from 250 bar to 800 bar. In parallel, the temperature applied to the dairy composition during the high-pressure homogenization step ranges from 60°C to 70°C, more preferably from 62 °C to 68 °C. The step of high-pressure homogenization contributes to increase the viscosity of the final product. For example, the high-pressure homogenization may be performed using a high-pressure homogenizer marketed by SPX such as the high-pressure homogenizer Rannie c5/Gaulin 5. Before reaching the outlet of the high-pressure homogenization device, the dairy composition may undergo a second step of homogenization with a temperature ranging from 62 °C to 68 °C and with a pressure ranging from 50 and 150 bar. This second step prevents a new aggregation of fat globules in the dairy composition.

After the high-pressure homogenization step, the homogenized dairy composition is pasteurized to obtain a pasteurized dairy composition. The pasteurization prevents any microbiological hazard by limiting the pathogenic microorganism load in the final product. The pasteurization is performed using a heat treatment with a temperature ranging from 85°C to 95°C for a time ranging from 3 minutes to 8 minutes. For example, the pasteurization treatment may be carried out in an indirect manner by means of a heat-plate exchanger. As a variant, it is possible to carry it out in a jacketed holding unit.

The pasteurized dairy composition is thereafter cooled down to a temperature between 32°C and 36°C, preferably between 32°C and 35°C. The said pasteurized dairy composition is then inoculated with a mesophilic strain as starter culture. The term mesophilic strains refers to a microorganism having an optimal growth at a temperature between 25°C and 35°C while the term thermophilic strain refers to a microorganism having an optimal growth at a temperature between 36°C and 45°C. The temperature where the strains have an optimal growth, namely optimum growth temperature, may be determined by methods known in the art, such as the method of Kanasaki et al. (1975) as provided for example in Ahmed et al., Influence of temperature on growth pattern of Lactococcus lactis, Streptococcus cremoris and Lactobacillus acidophilus isolated from camel milk, 2006, Biotechnology, Volume 5 (4): 481-488. Mesophilic strains are preferred to thermophilic strains because mesophilic strains have lower post-acidifying properties. Preferably, the pasteurized dairy composition is not inoculated with thermophilic strains. More preferably, thermophilic strains are not used in the claimed process. Hence, the fermented dairy product is substantially free, preferably entirely free, from thermophilic strains. In the context of the invention, the term "post-acidifying properties" refers to the ability of lactic strains to reduce the pH of a fermented dairy product containing between 2% and 4% of protein content over 28 days of storage at 8°C after the twenty-four hours following the end of the fermentation. The post-acidifying property is measured by calculating the pH drop: delta pH (ΔpH). Delta pH is the difference between the pH of the fermented dairy product containing between 2% and 4% of protein content measured at 8°C twenty-four hours after the end of the fermentation and the pH of the same fermented dairy product measured after 28 days of storage at 8°C after then end of fermentation.

$$\Delta pH = pH(24h) - pH(28days)$$

More particularly, the inoculated mesophilic strain has low post-acidifying properties. In the context of the invention, the term "low post-acidifying properties" means that the enzymatic activity of the strain leads to less than 0.22 pH units drop in a fermented dairy product containing between 2% and 4% of protein over 28 days of storage at 8°C after the twenty-four hours following the end of the fermentation, i.e. ΔpH ≤ 0.22. Fermented dairy products prepared with strains having low post-acidifying properties exhibit a limited acidity perceived in mouth. Indeed, limiting post-acidification enables to prevent the development of acidity in the fermented dairy product due to pH drop.

The mesophilic strain having low post-acidifying properties is selected from the group consisting of *Lactococcus lactis* subsp. *lactis diacetylactis* NCC 2216 having the accession number CNCM I-5134, *Lactococcus lactis* subsp. *lactis diacetylactis* NCC 2272 having the accession number CNCM I-5258, *Lactococcus lactis* subsp. *Lactis* NCC 2471 having the accession number NCIMB 8586 and *Lactococcus lactis* subsp. *Lactis* NCC 2205 having the accession number CNCM I-5257. Preferably, the mesophilic strain having low post-acidifying properties is *Lactococcus lactis* subsp. *lactis diacetylactis* NCC 2216 having the accession number CNCM I-5134.

*Lactococcus lactis* subsp. *lactis diacetylactis* NCC 2216 was deposited by Nestec S.A. with the accession number CNCM I-5134 on 13 September 2016, with the Collection Nationale de Cultures de Microorganismes (CNCM), Institut Pasteur, 25 Rue du Docteur Roux, F-75724 Paris Cedex 15, France, under the Budapest Treaty.

*Lactococcus lactis* subsp. *lactis diacetylactis* NCC 2272 and *Lactococcus lactis* subsp. *Lactis* NCC 2205 were desposited by Nestec S.A. respectively with the accession number CNCM I-52578 and CNCM I-5257 on 15 November 2017, with the Collection Nationale de Cultures de Microorganismes (CNCM), Institut Pasteur, 25 Rue du Docteur Roux, F-75724 Paris Cedex 15, France, under the Budapest Treaty.

The applicant Société des Produits Nestlé S.A. (SPN) is the successor in title of the depositor Nestec S.A..

*Lactococcus lactis* subsp. *lactis* NCC 2471 is available under the accession number 8586 with National Collection of Industrial Food and Marine Bacteria (NCIMB) Ltd,. Ferguson Building, Craibstone Estate, Bucksburn, Aberdeen, AB21 9YA, United Kingdom. *Lactococcus lactis* subsp. *Lactis* NCC 2471 is also available under the accession DSM 20729 with Deutsche Sammlung von Mikroorganismen und Zellkulturen (DSMZ) GmbH, Inhoffenstraße 7B, 38124 Braunschweig, Germany and under the accession number 11454 with American Type Culture Collection (ATCC), 10801 University Boulevard, Manassas, VA 20110, United States of America. *Lactococcus lactis* subsp. *Lactis* NCC 2471 is known in the art. For example, *Lactococcus lactis* subsp. *Lactis* NCC 2471 was studied in Tanigawa et al, Identification and typing of Lactococcus lactis by Matrix-Assisted Laser Desorption Ionization-Time of Flight Mass Spectrometry, Applied and environmental microbiology, June 2010, volume 76, n°12, p. 4055-4062. *Lactococcus lactis subsp. Lactis* NCC 2471 was also studied in Zacharof et al., Partially chemically defined liquid medium development for intensive propagation of industrial fermentation lactobacilli strains, Annals of Microbiology, 2013, Volume: 63, Issue: 4, Pages: 1235 - 1245; Zacharof et al., Separation of lactobacilli bacteriocins from fermented broths using membranes, Process Biochemistry, August 2013, Volume 48, Issue 8, Pages 1252-1261. Olasupo et al., Occurrence of nisin Z production in Lactococcus lactis BFE 1500 isolated from wara, a traditional Nigerian cheese product, Int J Food Microbiol., December 1999, 15;53(2-3):141-52.

The step of inoculation may be a step of direct inoculation or a step of semi-direct inoculation. Direct inoculation is a method where the strain is directly added in the pasteurized dairy composition. Contrarily, semi-direct inoculation is a method where the strain is first inoculated in a bulk starter to promote the growth of the strain. After a preliminary growth of the strain into the bulk starter, the strain is inoculated in the pasteurized dairy composition. A person having ordinary skill in the art will choose between semi-direct inoculation and direct inoculation depending on the nature of the strain raw material and on the suppliers' recommendations.

The inoculated dairy composition is then fermented at a temperature between 32°C and 36°C, preferably between 32°C and 35°C, until a pH ranging from 4.5 to 5.5 is reached to obtain a fermented dairy product.

After reaching the target pH of from 4.5 to 5.5, the fermented dairy product may be optionally smoothed and cooled down to a temperature between 10°C and 25°C.

A warm flavouring agent is mixed to the fermented dairy product after the fermentation step and/or to the dairy composition during the first step before the homogenization step. When the fermented dairy product is a set yoghurt, the warm flavouring agent is mixed to the dairy composition during the first step before the homogenization step. The term "warm" flavouring agent is used to designate ingredients providing the flavour of for example chocolate, caramel, vanilla, coffee, praline, biscuit, nougat, cinnamon and/or the flavour of oleaginous fruits (peanut, walnut, hazelnut, almond, pistachio nut, cashew nut), and the like. Especially, the term "warm" associated to a flavour/flavouring agent refers to flavours/flavouring agents which are not acidic and fruity. The term "warm" associated to a flavour/flavouring agent is a well-known and common term in the sensory field. Especially, it is commonly used in the sensory assessment of the flavour profile of food products or food ingredients. The warm flavouring agent of the present invention is selected from the group consisting of chocolate flavouring agent, vanilla flavouring agent, coffee flavouring agent, biscuit flavouring agent, cinnamon flavouring agent, caramel flavouring agent, praline flavouring agent, nougat flavouring agent, peanut flavouring agent, hazelnut flavouring agent, pistachio flavouring agent, almond flavouring agent, walnut flavouring agent, cashew nut flavouring agent and mixtures thereof. The warm flavouring agent may be an artificial flavouring agent or a non-artificial flavouring agent. In the context of the invention, the term "artificial flavouring agent" refers to a molecule or a combination of molecules, the function of which is to impart flavour, obtained by chemical synthesis. Contrarily, the term "non-artificial flavouring agent" refers to flavouring agents which are not artificial flavouring agent. This term includes, naturally-occurring molecules or preparations (e.g juice, purée...) extracted from plant materials present in nature (e.g spices, fruits, vegetables, herbs, barks, buds, roots, leaves, meats, fishes, poultries, eggs, dairy products) and fermentation products. Examples of non-artificial flavouring agent include for instance caramel sauce or chocolate sauce obtained after heating, respectively sugar or chocolate. The warm flavouring agent may be in a form of a liquid (e.g. artificial caramel flavour concentrate, caramel sauce, chocolate sauce), a paste (e.g. hazelnut paste, pistachio paste), a powder (e.g. cocoa powder) or solid inclusions (e.g. chocolate chips). In a preferred embodiment, the warm flavouring agent is a liquid non-artificial warm flavouring agent. In a more preferred embodiment, the warm flavouring agent is a caramel sauce or a chocolate sauce. The chocolate sauce may be a dark chocolate sauce, a milk chocolate sauce or a white chocolate sauce. Preferably, the chocolate sauce is a dark chocolate sauce. The warm flavouring agent content will vary depending upon the type of the warm flavouring agent used and the warm flavouring agent intensity desired in the final product.

In an embodiment, the fermented dairy product may be whipped to obtain an aerated fermented dairy product. The overrun of the aerated fermented dairy product may be between 30% and 200%. Preferably, the overrun of the aerated fermented dairy product is between 75% and 170%. A standard aeration mixer may be used for whipping the fermented dairy product. Suitable equipment includes aeration mixers supplied by AEROMIX or MONDOMIX. Another suitable aeration mixer is the one disclosed in the patent EP 2 775 853 B1. The fermented dairy product is aerated using a food grade gas. Examples of food grade gases include nitrogen, air and carbon dioxide. Nitrogen is preferred.

The fermented dairy product containing at least one warm flavouring agent is thereafter cooled down and stored to a temperature of 8°C.

In an embodiment of the invention, the egg-and-milk powder is prepared by first mixing, while stirring, a concentrated lactic base rich in milk proteins with liquid egg to obtain an egg-milk liquid mixture.

The liquid egg may be a mixture of egg yolk and egg white, or preferably a whole egg in liquid form, which has been pasteurized beforehand. Fresh egg or ripened egg may be used. In the context of the invention, ripened egg refers to an egg which has been stored at refrigeration temperature, for example at about 4°C, for a period of up to several days. The liquid egg may be a mixture of egg whites ripened separately from egg yolks. The liquid egg may be a mixture of fresh egg whites and/or ripened egg whites and/or fresh egg yolks and/or ripened egg yolks. The liquid eggs may be whole eggs mixed with ripened egg yolks and/or fresh egg yolks and/or ripened egg whites and/or fresh egg whites. All the raw materials contained in the liquid egg are separately pasteurized.

The liquid egg of the egg-and-milk powder is preferably a low temperature pasteurized whole egg. Low temperature pasteurization of egg, in the context of the present invention, refers to a pasteurization performed with a temperature, which is between 62°C and 72°C, preferably between 64°C and 69°C for a time ranging from 30 seconds to 150 seconds. In general, a temperature between 64°C and 69°C, refers to a temperature at which the egg proteins contained in eggs do not coagulate. For undiluted eggs or eggs not supplemented with additives, this temperature is considered to be about 65.5 °C.

In the context of the invention, the expression lactic base rich in milk protein is understood to mean any raw material of lactic origin containing from 35% to 80% by weight of plant proteins or whey proteins, more especially lactalbumins. Examples of plant proteins include pulse proteins and soy proteins. By lactic origin, it is understood that the raw material is derived either from a non-human mammal milk or from a plant milk. Preferably, the lactic base rich in milk protein is a whey protein concentrate containing from 35% to 80% by weight of whey proteins. Such a whey protein concentrate may be obtained from the ultrafiltration or the microfiltration of sweet or acid whey from casein making or from cheese making from whey. The concentrate may be demineralized or made lactose free.

Without wishing to be bound by theory, the inventors believe that whey proteins such as lactalbumins are preferred because they have a structure similar to the structure of egg proteins such as ovalbumins. Due to a similar structure, the whey proteins easily interact with the egg proteins to form whey protein/egg protein aggregates. Therefore, the whey proteins by interacting with the egg proteins, protect the egg proteins and prevent the coagulation between the egg proteins during the different heat-treatments along the manufacturing process.

The pH of the egg-milk liquid mixture is then adjusted, where appropriate, to a value ranging from 6.5 to 7.5. More particularly, when the pH is over 7.5, the pH is decreased using a food grade acidic solution to reach a pH between 6.5 and 7.5. Examples of food grade acidic solutions include lactic acid, hydrochloric acid, phosphoric acid and citric acid. When the pH is below 6.5, the pH is increased using a food grade alkaline solution to reach a pH between 6.5 and 7.5. An example of a food grade alkaline solution is sodium hydroxide.

After pH adjustment, the egg-milk liquid mixture is pasteurized. The pasteurization treatment may be carried out in an indirect manner by means of a plate-heat exchanger. As a variant, the pasteurization treatment may be carried out in a jacketed holding unit. The pasteurization should be carried out under conditions which are lethal for *Salmonella* while preventing coagulation of the egg, More particularly, the egg-milk liquid mixture is pasteurized at a temperature ranging from 64°C to 75°C for a time ranging from 5 seconds to 150 seconds, more particularly at a temperature ranging from 70°C to 75°C for a time ranging from 10 seconds to 100 seconds. More particularly, the egg-milk liquid mixture is pasteurized at a temperature ranging from 70°C to 75°C for a time ranging from 5 seconds to 50 seconds. More particularly, the egg-milk liquid mixture is pasteurized at a temperature ranging from 70°C to 75°C for a time ranging from 5 seconds to 20 seconds. A person having ordinary skill in the art will select a couple temperature and time that prevents the egg in the egg-milk liquid from coagulating while significantly reducing the load of *Salmonella* to a quantity where there is no more health concern for the human. Classically, the higher the temperature selected is, the lower the time selected is.

Preferably, the egg-milk liquid mixture is pasteurized at a temperature of 72°C for 15 seconds.

The pasteurized egg-milk liquid mixture is dried afterwards to obtain an egg-and-milk powder. For example, the pasteurized mixture may be spray-dried in a drying tower using an entry temperature of 185°C, an exit temperature of 90°C and a turbine rotation of 20000 tr/min.

The egg-and-milk powder is more convenient than an egg or an egg-milk composition in liquid form. The egg-and-milk powder is stable for a long period from the microbiological point of view. In addition, the egg-and-milk powder is not susceptible to recontamination, contrary to an egg or an egg-milk composition in liquid form. Furthermore, the egg-and-milk powder does not undergo phase separation or sedimentation during storage or upon reconstitution.

Without wishing to be bound by theory, in the case of the egg-and-milk powder, the inventors believe that the milk or whey proteins protect the egg proteins from coagulation at a temperature above 64°C. This protection may account for the ability to conduct a pasteurization even above 70°C, which means that hygiene criteria for many uncooked products are met, while at the same time the functional properties of egg are not lost. The use of eggs which have been simply pasteurized (e.g pasteurized liquid egg) in a conventional manner under conditions which maintain their organoleptic and functional properties would not be possible in uncooked food products such as fermented dairy products. Indeed, hygiene requirements would not be guaranteed.

In a preferred embodiment, the egg-and-milk powder is obtained without addition of stabilizing or emulsifying additives, and without adding water before pasteurization.

The egg-and-milk powder may also be prepared using the process disclosed in the patent application WO 2002/07541 A1. The egg-and-milk powder is known to have texturizing and emulsifying properties in the state of the art (e.g. WO 2002/07541 A1). However, the inventors have surprisingly discovered that the egg-and-milk powder, beyond its texturizing and emulsifying properties, enables to lower the perceived acidity in mouth efficiently and significantly. The use of the egg-and-milk powder in the process of the invention is then key to obtaining a fermented dairy product matching with warm flavouring agents.

In a second aspect, the invention relates to a fermented dairy product obtainable by the process disclosed in the first aspect. The fermented dairy product may be a drinkable fermented milk, a quark, a drinkable yoghurt or a spoonable yoghurt. Preferably, the fermented dairy product is a spoonable yoghurt. When the fermented dairy product is a spoonable yoghurt, it may be a set-style yoghurt, a stirred yoghurt or a Greek-style yoghurt.

In a particular embodiment, the fermented dairy product consists of a whipped fermented dairy product. In such an embodiment, the whipped fermented dairy product has an overrun ranging from 30% to 200%, more preferably from 75% to 170%. When aerated, a fermented dairy product exhibits a decrease in acidity perceived in mouth.

The pH of the fermented dairy product is acidic. More especially, the fermented dairy product has a pH between 4.5 and 5.5, and more preferably between 4.5 and 5.0. Contrary to neutral desserts, a fermented dairy product with acidic pH is advantageous in terms of hygiene and microbiological spoilage concerns. Indeed, an acidic pH is less favourable than a neutral pH for the development of pathogenic microorganisms. In spite of an acidic pH, the fermented dairy product of the invention exhibits a low acidity perceived in mouth in order to match with warm flavouring agents.

The fermented dairy product has a protein content ranging from 2% to 4% based on the total weight of the fermented dairy product, preferably, ranging from 2% to 3% based on the total weight of the fermented dairy product. The protein in the fermented dairy product may be milk proteins, egg proteins, plant proteins and/or meat proteins. This low protein content enables to limit the buffering effect of proteins. The higher the protein content, the higher the content in acidic molecules such as lactic acid is required to reach a targeted pH value during fermentation. The higher the amount of lactic acid, the higher the Dornic acidity is. A too high content in acidic molecules and a too high Dornic acidity results in an acidity perceived in mouth which is too strong to match with warm notes provided by warm flavouring agents. The protein content of the invention is chosen to limit as much as possible the buffering effect of proteins in order to have a low acidity perceived in mouth. When the protein content is higher than 4 wt%, the content in acidic molecules and the Dornic acidity are too high. This results in a fermented dairy product having an acidity perceived in mouth that does not match with warm notes provided by warm flavouring agents. When the protein content is lower than 2wt%, the protein content is insufficient to form a satisfactory gel during fermentation. The resulting texture of the fermented dairy product is therefore too low (i.e. too liquid, not enough thick) in the case where the fermented dairy product is a yoghurt or a drinkable yoghurt.

As also mentioned above in connection with the description of the manufacturing process, the fermented dairy product comprises a mesophilic strain. the mesophilic strain having low post-acidifying properties is selected from the group consisting of: *Lactococcus lactis* subsp. *lactis diacetylactis* NCC 2216 having the accession number CNCM I-5134, *Lactococcus lactis* subsp. *lactis diacetylactis* NCC 2272 having the accession number CNCM I-5258, *Lactococcus lactis* subsp. *Lactis* NCC 2471 having the accession number NCIMB 8586 and *Lactococcus lactis* subsp. *Lactis* NCC 2205 having the accession number CNCM I-5257. Preferably, the mesophilic strain having low post-acidifying properties is *Lactococcus lactis* subsp. *lactis diacetylactis* NCC 2216 having the accession number CNCM I-5134. In a preferred embodiment, the fermented dairy product is substantially free, preferably entirely free from thermophilic strains.

The fermented dairy product comprises at least one warm flavouring agent. The warm flavouring agent is selected from the group consisting of chocolate flavouring agent, vanilla flavouring agent, coffee flavouring agent, biscuit flavouring agent, cinnamon flavouring agent, caramel flavouring agent, praline flavouring agent, nougat flavouring agent, peanut flavouring agent, hazelnut flavouring agent, pistachio flavouring agent, almond flavouring agent, walnut flavouring agent, cashew nut flavouring agent and mixtures thereof. The warm flavouring agent may be an artificial flavouring agent or a non-artificial flavouring agent. The warm flavouring agent may be in a form of a liquid (e.g. artificial caramel flavour concentrate, caramel sauce, chocolate sauce), a paste (e.g. hazelnut paste, pistachio paste), a powder (e.g. cocoa powder) or solid inclusions (e.g. chocolate chips). In a preferred embodiment, the warm flavouring agent is a liquid non-artificial warm flavouring agent. In a more preferred embodiment, the warm flavouring agent is a caramel sauce or a chocolate sauce. The chocolate sauce may be a dark chocolate sauce, a milk chocolate sauce or a white chocolate sauce. Preferably, the chocolate sauce is a dark chocolate sauce. The warm flavouring agent content will vary depending upon the type of the warm flavouring agent used and the warm flavouring agent intensity desired in the final product.

In another embodiment, the fat content of the fermented dairy product is up to 5%, preferably 3% based on the total weight of the fermented dairy product.

In a further embodiment, the fermented dairy product has a Dornic acidity ranging from 60 °D to 80 °D.

In a third aspect, the invention relates to a multilayer food product comprising a fermented dairy product according to the invention. The multilayer food product contains at least two layers. The multilayer food product comprises at least one layer of a first fermented dairy product according to the invention comprising a first warm flavouring agent and at least one layer consisting of either a dessert preparation or a second fermented dairy product according to the invention comprising a warm flavouring agent different from the first warm flavouring agent. The dessert preparation may be a whipped cream, a fruit preparation, a cereal preparation, a fruit jelly, a fresh cheese, a fruit-flavoured yoghurt, a plain yoghurt, a plain yoghurt mousse, a fruit mousse or a panna cotta. When the multilayer food product comprises two layers, the fermented dairy product according to the invention may be a top layer or a bottom layer. When the multilayer food product comprises at least three layers, the fermented dairy product according to the invention may be a top layer, a bottom layer or an intermediate layer.

In a fourth aspect, the invention relates to a mesophilic strain with low post-acidifying properties selected from the group consisting of *Lactococcus lactis* subsp. *lactis diacetylactis* NCC 2216 having the accession number CNCM I-5134, *Lactococcus lactis* subsp. *lactis diacetylactis* NCC 2272 having the accession number CNCM I-5258 and *Lactococcus lactis* subsp. *Lactis* NCC 2205 having the accession number CNCM I-5257. Especially, such mesophilic strains have not only low post-acidifying properties but are suitable for use in a food product comprising a warm flavouring agent. Preferably, the food product is a fermented dairy product. More preferably, the food product is a fermented dairy product as described in the first, second and third aspect of the invention.

Further details about the warm flavouring agents are provided in the first, second and third aspects of the invention.

By "suitable for use in a food product comprising a warm flavouring agent", it is understood that the mesophilic strain generates a flavour profile within the food product, especially after fermentation, that matches with warm notes provided by the warm flavouring agents. Especially, the mesophilic strain generates a low acidity perceived in mouth. As previously indicated in the specification, the acidity perceived in mouth may be assessed by trained persons (e.g a trained panel) using a ranking from 1 (i.e. not acid, neutral) to 10 (very acid). Preferably, the food product has an acidity perceived in mouth with a ranking ranging from 1 to 5, more preferably from 1 to 3. The inventors have discovered that all mesophilic strains with low post-acidifying properties are not suitable for use in food products comprising warm flavouring agent. Only specific mesophilic strains with low post-acidifying properties are able to generate a flavour profile within a food product, especially a low acidity perceived in mouth, that matches with warm notes provided by warm flavouring agents. Especially, the inventors have discovered that the three strains NCC 2216, NCC 2272 and NCC 2205 are able to generate a flavour profile within a food product, especially a fermented food product, that matches with warm notes provided by warm flavouring agents.

## EXAMPLES

### Example 1: Assessment of post-acidifying properties of strains

Material & Methods:

Post acidifying properties of the following four mesophilic strains were studied:

- *Lactococcus lactis* subsp. *lactis diacetylactis* having the accession number CNCM I-5134 (NCC 2216),
- *Lactococcus lactis* subsp. *lactis diacetylactis* having the accession number CNCM I-5258 (NCC 2272),
- *Lactococcus lactis* subsp. *lactis* having the accession number NCIMB 8586 (NCC 2471),
- *Lactococcus lactis* subsp. *lactis* having the accession number CNCM I-5257 (NCC 2205).

A semi-direct inoculation was performed. More particularly, 0.5mL of frozen concentrate ($5x10^9$ cfu/ml) of each mesophilic strain (NCC 2216, NCC 2272, NCC 2471 and NCC 2205) was inoculated separately into 500 mL bulk starter enriched with 0.1 wt% yeast extract. The inoculated bulk starter was then fermented in a 34°C water-bath until a pH of 4.8 is reached.

In parallel, a whole UHT milk (3.2wt% protein) was pasteurized at 95°C for 5 minutes. Thereafter, the pasteurized milk was cooled down to 5°C and warmed up to 34°C.

Thereafter, the pasteurized milk was inoculated with the bulk starter to obtain an inoculated milk and then filled into polystyrene cups. Thereafter, the inoculated milk was incubated at 34°C until pH 4.6 is reached. At the end of fermentation, the cups were placed into a blast cooler for rapid cooling and then kept at 8°C for 28 days for analysis.

The pH of each preparation was measured twenty-four hours after the end of the fermentation at 8°C and after 28 days of storage at 8°C after the end of fermentation. The pH drop (delta pH) was determined by calculating the difference between the pH of the preparation measured twenty-four hours after the end of the fermentation at 8°C and the pH of the preparation measured after 28 days of storage at 8°C after the end of the fermentation.

Results:

The results presented in Figure 1 show that the four tested strains (NCC 2216, NCC 2272, NCC 2471 and NCC 2205) have low post-acidifying properties (cf. Figure 1). More especially, NCC 2216 has the lowest post-acidifying property with a pH drop of less than 0.1 unit after 28 days of storage in milk. Therefore, these four strains are promising candidates to limit the acidity perceived in mouth of fermented dairy products.

**Example 2: Lab scale trials**

Material and methods:

Set yoghurts were prepared at laboratory scale.

Three samples, sample 1 to sample 3, were prepared using the following recipe: 94% UHT full-fat milk, 4% sucrose and 2% skimmed milk powder

Six other samples, sample 4 to sample 9, were prepared using the following recipe: 94% UHT full-fat milk, 4% sucrose and 2% egg-and-milk powder.

After mixing the ingredients listed above, the samples were sterilized by immersion of the container containing the samples in a boiling water-bath, until the inner product temperature reached 95°C. Thereafter, the samples were cooled down to 6°C by immersion into a cold water-bath.

Prior to their inoculation, the samples were pre-heated to the optimal temperature ranging from 34°C to 40°C, depending on the strains used (cf. Fermentation temperature in Table 1).

A first set of samples, sample 1 to sample 6, was inoculated and fermented according to the conditions disclosed in Table 1, without any added flavours (cf. Table 1). After fermentation, sample 1 to sample 6 were cooled and stored at 8°C.

Strain A and Strain B (cf. Table 1 and Table 2) are thermophilic strains from the Nestlé collection. Strain A is a combination of two *Streptococcus thermophilus* strains. Strain B is a *Lactobacillus delbrueckii* subsp. *Bulgaricus* strain. They were added in combination with mesophilic strains or not, to assess the impact of thermophilic strains on the acidity perceived in mouth compared to the impact of mesophilic strains alone on the acidity perceived in mouth.

Samples 1 to 6 were not mixed with a warm flavouring agent. However, the assessment of the acidity perceived in mouth for samples 1 to 6 enabled to determine whether the samples is suitable to match with warm flavouring agent. Indeed, the lower the acidity perceived in mouth is, the better the sample matches with warm flavouring agent.

Table 1

| Sample | Type of powder | Strain A | Strain B | NCC 2471 | Fermentation temperature |
|---|---|---|---|---|---|
| 1 | 2% Skimmed milk powder | 2% | 1% | No | 40°C |
| 2 | | 1.5% | 0.5% | 1% | 36°C |
| 3 | | no | No | 2% | 34°C |

(continued)

| Sample | Type of powder | Strain A | Strain B | NCC 2471 | Fermentation temperature |
|---|---|---|---|---|---|
| 4 | | 2% | 1% | no | 40°C |
| 5 | 2% Egg-and-milk powder | 1.5% | 0.5% | 1% | 36°C |
| 6 | | no | No | 2% | 34°C |

A second set of samples, sample 7 to sample 9, was inoculated and fermented according to the conditions disclosed in Table 2, with 0.6wt% of an artificial caramel flavour (cf. Table 2). After fermentation, sample 7 to sample 9 were cooled and stored at 8°C.

Table 2

| Sample | Type of powder | Strain A | Strain B | Mesophilic Strains | Fermentation temperature |
|---|---|---|---|---|---|
| 7 | | 2% | 1% | no | 40°C |
| 8 | 2% Egg-and-milk powder | 1.50% | 0.50% | 1% NCC 2205 | 36°C |
| 9 | | 1.50% | 0.50% | 1% NCC 2216 | 36°C |

The pH of each sample was measured twenty-four hours after the end of the fermentation (Final pH) at 8°C and 14 days after the end of the fermentation (pH+D14) at 8°C. The difference between the two pH measures provides the decrease in pH in the samples after 14 days. This decrease in pH in the samples after 14 days is equivalent to the pH drop (delta pH) as defined in example 1. Indeed, based on their experience, the inventors have found that the pH drop at 14 days after the end of fermentation, and the pH drop at 28 days after the end of the fermentation are the same.

Results:

Fermentation times used and pH values measured for each sample are indicated in Table 3 below:
The results confirm that the decrease in pH after 14 days of shelf-life (pH+D14) is significantly lower when pure mesophilic strains are used, in comparison with pure thermophilic strains. In the latter case, the decrease in pH after 14 days of shelf-life (ie the difference between the final pH and pH+D14) is higher than 0.2 pH units. Similarly, as long as mesophilic strains are used, either in pure culture or in combination with thermophilic strains, and partially because incubation temperature has to be lowered to allow its growth, the fermentation times are significantly longer.

Finally, five persons trained to describe the acidity perceived in mouth tasted the products.

Despite a significantly different pH value measured between samples 1 and 4 (0.15 pH units less for the sample 4 containing egg-and-milk powder), no acidity difference was perceived in mouth from a sensory point of view during tastings. The presence of egg-and-milk powder seems to help in decreasing the acidity in perceived mouth. Nevertheless, it cannot be concluded if this decrease in acidity perception is linked to a difference in buffering capacity between the egg proteins and the milk proteins, or linked to the presence of specific compounds in the egg-and-milk powder that positively impact on the acidity perception. The fact that samples containing the egg-and-milk powder are slightly richer in fat, 2% of egg-and-milk powder increasing by 0.5% the fat content in the final product, could help in decreasing acidity perception in mouth. Nevertheless, we cannot exclude whether this effect is attributable to flavour compounds having an acidity masking effect.

Moreover, tastings show that samples containing the egg-and-milk powder have a lower acidity perceived in mouth compared to samples containing the skimmed milk powder. More especially, the best performing sample was the one containing a pure mesophilic strain (NCC 2471) and egg-and-milk powder, namely, sample 6. The combination of the mesophilic strains and the egg-and-milk powder appears to be a good strategy to achieve a low acidity perceived in mouth.

Table 3

| Sample | Type of powder | Strains | Fermentation temperature | Incubation Times | Final pH | pH+D14 |
|---|---|---|---|---|---|---|
| 1 | Skimmed milk powder | Strain A/Strain B | 40°C | 7h45 | 4.6 | 4.38 |
| 2 | | Strain A/Strain B /NCC 2471 | 36°C | 24h00 | 4.38 | 4.33 |
| 3 | | NCC 2471 | 34°C | 24h00 | 4.45 | 4.47 |

(continued)

| Sample | Type of powder | Strains | Fermentation temperature | Incubation Times | Final pH | pH+D14 |
|---|---|---|---|---|---|---|
| 4 | Egg-and-milk powder | Strain A/Strain B | 40°C | 6h50 | 4.47 | 4.23 |
| 5 | | Strain A/Strain B /NCC2471 | 36°C | 9h10 | 4.64 | 4.34 |
| 6 | | NCC 2471 | 34°C | 24h00 | 4.5 | 4.43 |
| 7 | Egg-and-milk powder | Strain A/Strain B | 40°C | 5h50 | 4.51 | 4.39 |
| 8 | | Strain A/Strain B /NCC 2205 | 36°C | 6h40 | 4.57 | 4.45 |
| 9 | | Strain A/Strain B /NCC 2216 | 36°C | 8h00 | 4.64 | 4.55 |

**Example 3: Bench scale trials**

Material & Methods:

Stirred yoghurts (both caramel-flavoured and chocolate-flavoured) were prepared at bench scale. Bench scale refers to facilities which enable the processing of from 20L to 90L of product per hour. A comparison was done between an enrichment with 2% skimmed milk powder and an enrichment with 2% egg-and-milk powder. The recipe of the preparation containing 2% skimmed milk powder is indicated in table 4 below:

Table 4

| Ingredients (Recipe with skimmed milk powder) | % |
|---|---|
| Skimmed milk | 81.0 |
| Cream 34% Fat | 13.0 |
| Sugar | 4.0 |
| Skimmed milk powder | 2.0 |

Since the egg-and-milk powder contains about 26% fat, a small adjustment was done with cream to get the same fat content in both finished products. The recipe of the preparation containing 2% egg-and-milk powder is indicated in table 5 below:

Table 5

| Ingredients (Recipe with egg-and-milk powder) | % |
|---|---|
| Skimmed milk | 82.5 |
| Cream 34% Fat | 11.5 |
| Sugar | 4.0 |
| Egg-and-milk powder | 2.0 |

After mixing the ingredients of each preparation, each preparation was submitted to an upstream high-pressure homogenization (65°C / 700bar / 100bar), and pasteurized at 92°C for 6 minutes. The pasteurized preparations were cooled down to 34°C, inoculated with 1.5% NCC 2216 bulk starter and incubated at 34°C for 16H. Then, the preparation containing egg milk powder was split into two preparations: one added with 20% of a chocolate sauce and one added with 15% of a caramel sauce.

The preparation containing skimmed milk powder was mixed with 15% of a caramel sauce. Finally, the three preparations were then stored at 8°C.

The pH of each preparation was measured:

- after the end of the fermentation before adding the chocolate or caramel sauce (pH after 16H),
- 24 hours after the end of the fermentation (pH D+1),
- 28 days after the end of the fermentation (pH D+28).

Results:

pH values measured are indicated in the table 6 below:

Table 6

| Type of powder | pH after 16H | Type of sauce | pH D+1 | pH D+28 |
|---|---|---|---|---|
| 2% skimmed milk powder | 4.70 | 15% caramel | 4.83 | 4.75 |
| 2% egg-and-milk powder | 4.57 | 15% caramel | 4.70 | 4.61 |
| 2% egg-and-milk powder | 4.57 | 20% chocolate | 4.76 | 4.66 |

As previously observed, we noticed that lower pH values could be reached within a given period of incubation time in the presence of the egg-and-milk powder.

The addition of caramel or chocolate sauces leads to a pH increase (0.1 and 0.14 units respectively). Consequently, due to the very low post-acidification characteristics of NCC 2216, the pH of final product remains higher than 4.60 all along the shelf-life.

Finally, tastings of the different preparations with five persons trained to describe the acidity perceived in mouth were performed.

Concerning the two preparations containing the caramel sauce, despite the fact that the pH of the preparation containing egg-and-milk powder was the lowest (pH 4.61); it was perceived as being less acidic than the preparation with skimmed milk powder (pH 4.75). Apart from being perceived less acidic, which allows to get a better balance with the caramel flavour, the preparation with the egg-and-milk powder offered a better mouthfeel, a more indulgent texture, compared to the preparation with the skimmed milk powder.

**Example 4: Aerated fermented dairy product**

Two whipped fermented caramel samples were prepared, using the same recipe and the same process. The two samples were prepared by mixing the ingredients of Table 7 below.

Table 7

| Ingredient | % |
|---|---|
| Skimmed milk | 65.54 |
| Cream 34% fat | 25.00 |
| Sugar | 3.50 |
| Monoglyceride Dimodan HP | 0.48 |
| Emulsifier Lactem PQ22 | 0.48 |
| Skimmed milk powder | 1.50 |
| Egg-and-milk powder | 3.50 |

After mixing of the ingredients, the samples were then submitted to an upstream pressure homogenization (65°C/300 bar) and pasteurized at 92°C for 6 minutes. The samples were cooled down to 34°C, inoculated with 1.5% NCC 2216 bulk starter and incubated at 34°C for 16h-18h (targeted final pH is 4.60). Then, the samples were added with 15% of a caramel sauce. The whipping step was performed to reach various overrun levels: 75%, 110%, 130% and 160%.

Results:

Tastings of the different samples with five persons trained to describe the acidity perceived in mouth were performed.

From a sensory point of view, the product having 110% overrun was the one with the best balance between caramel flavour and sweetness perception. Moreover, tastings shows that the higher the overrun, the lower the acidity perceived in mouth is. Therefore, aeration enable to limit acidity perceived in mouth of fermented dairy products.

**Example 5: Assessment of acidification properties of strain NCC 2057**

The acidification properties of a mesophilic strain: *Lactococcus lactis subsp. lactis biovar diacetylactis* Sd28, also called strain NCC 2057 was assessed.

In a first step, a fermented dairy product was prepared according to the method disclosed in example 1 with the strain NCC 2057. The pH drop (delta pH) was determined according to the calculation disclosed in example 1.

A pH drop of 1.5 was calculated for the fermented dairy product prepared with strain NCC 2057. Hence, strain NCC 2057 has low post-acidifying properties like the strains disclosed in example 1.

In a second step, fermented dairy products comprising a chocolate sauce was prepared with strain NCC 2057 or NCC 2272 or NCC 2471 or NCC 2216.

0.5mL of frozen concentrate ($5x10^9$ cfu/ml) of mesophilic strain NCC 2057, NCC 2272, NCC 2471 or NCC 2216 was inoculated separately into 500 mL bulk starter enriched with 0.1 wt% yeast extract. The inoculated bulk starter was then fermented in a 34°C water-bath until a pH of 4.8 is reached.

In parallel, 1L of whole UHT milk comprising 4.5wt% of added sucrose was pasteurized at 95°C for 5 minutes. Thereafter, the pasteurized milk was cooled down to 5°C and warmed up to 34°C.

Thereafter, the pasteurized milk (1L) was inoculated with 10mL of bulk starter to obtain an inoculated milk and then filled into polystyrene cups. Thereafter, the inoculated milk was incubated at 34°C until pH 4.6 is reached to obtain a fermented dairy product. At the end of fermentation, the cups were placed into a blast cooler for rapid cooling and then kept at 8°C. Twenty-four hours after the end of the fermentation, 15wt% of a chocolate sauce comprising 22wt% sucrose, 15wt% dark chocolate and 2wt% cocoa was added to the obtained fermented dairy product.

The obtained fermented dairy products comprising 15wt% of a chocolate sauce was assessed by five persons trained to describe the acidity perceived in mouth were performed.

Despite the low post-acidifying properties of strain NCC 2057, the fermented dairy product prepared with strain NCC 2057 has an unpleasant taste. Indeed, the obtained fermented dairy product has a significant acidity perceived in mouth leading to a flavor profile that does not match with the chocolate sauce.

In opposition, the fermented dairy products prepared with the mesophilic strains having low post-acidifying properties NCC 2272, NCC 2471 or NCC 2216 exhibit a low acidity perceived in mouth and their respective flavour profiles match with the chocolate sauce.

Hence, in opposition to the mesophilic strains having low post-acidifying properties discloses in examples 1 to 4, the mesophilic strain NCC 2057 is not suitable for use in a fermented dairy product comprising a warm flavouring agent such as a chocolate sauce.

Although the invention has been described by way of example, it should be appreciated that variations and modifications may be made without departing from the scope of the invention as defined in the claims.

**Claims**

1. A process for the preparation of a fermented dairy product having at least one warm flavouring agent, a pH between 4.5 and 5.5 and a protein content between 2% and 4% based on the total weight of the fermented dairy product comprising the steps of:

   a) preparing a dairy composition by mixing an egg-and-milk powder with a dairy base
   b) homogenizing the dairy composition by high-pressure homogenization at a pressure ranging from 250 bar to 800 bar and a temperature ranging from 60°C to 70°C to obtain an homogenized dairy composition, pasteurizing the homogenized dairy composition at a temperature ranging from 85°C to 95°C for a time ranging from 3 minutes to 8 minutes to obtain a pasteurized dairy composition,
   c) cooling the pasteurized dairy composition to a temperature between 32°C and 35°C, and then inoculating the pasteurized dairy composition with a mesophilic strain to obtain an inoculated dairy composition, wherein the mesophilic strain is a mesophilic strain having low post-acidifying properties, and wherein the mesophilic strain having low post-acidifying properties is from the genus *Lactococcus and* is selected from the group consisting of *Lactococcus lactis* subsp. *lactis diacetylactis* NCC 2216 having the accession number CNCM I-5134, *Lactococcus lactis* subsp. *lactis diacetylactis* NCC 2272 having the accession number CNCM I-5258, *Lactococcus lactis* subsp. *Lactis* NCC 2471 having the accession number NCIMB 8586 and *Lactococcus lactis* subsp. *Lactis* NCC 2205 having the accession number CNCM I-5257,
   d) fermenting the inoculated dairy composition at a temperature between 32°C and 35°C until a pH ranging from 4.5 to 5.5 is reached, to obtain a fermented dairy product,
   e) mixing at least one warm flavouring agent during step a) and/or after step d), wherein the warm flavouring agent is selected among the group consisting of chocolate flavouring agent, vanilla flavouring agent, coffee flavouring agent, biscuit flavouring agent, cinnamon flavouring agent, caramel flavouring agent, praline flavouring agent, nougat flavouring agent, peanut flavouring agent, hazelnut flavouring agent, pistachio flavouring agent, almond flavouring agent, walnut flavouring agent, cashew nut flavouring agent and mixtures thereof,

f) optionally, cooling the fermented dairy product containing said warm flavouring agent to a temperature of 8°C.

2. A process for the preparation of a fermented dairy product according to claim 1 wherein the egg-and-milk powder is prepared by:

a') mixing, while stirring, a concentrated lactic base rich in milk proteins with liquid egg to obtain an egg-milk liquid mixture

b') adjusting the egg-milk liquid mixture, where appropriate, to a value ranging from 6.5 to 7.5 c') pasteurizing the egg-milk liquid mixture at a temperature ranging from 64°C to 75°C for a time ranging from 5 seconds to 150 seconds

d') drying the pasteurized mixture to obtain an egg-and-milk powder

3. A process for the preparation of a fermented dairy product according to claim 2 wherein during step c'), the egg-milk liquid mixture is pasteurized at a temperature ranging from 70°C to 75°C for a time ranging from 10 to 100 seconds.

4. A process for the preparation of a fermented dairy product according to claim any one of claims 1 to 3, wherein the mesophilic strain having low post-acidifying properties has an enzymatic activity that leads to less than 0.22 pH units drop in a fermented dairy product containing between 2% and 4% of protein over 28 days of storage at 8°C after the twenty-four hours following the end of the fermentation.

5. A fermented dairy product having a pH between 4.5 and 5.5 and a protein content ranging from 2% to 4% based on the total weight of the fermented dairy product comprising a mesophilic strain and at least one warm flavouring agent, obtainable by the process of any one of claims 1 to 4, wherein the mesophilic strain is a mesophilic strain having low post-acidifying properties,

wherein the mesophilic strain is from the genus *Lactococcus* and is selected from the group consisting of *Lactococcus lactis* subsp. *lactis diacetylactis* NCC 2216 having the accession number CNCM I-5134, *Lactococcus lactis* subsp. *lactis diacetylactis* NCC 2272 having the accession number CNCM I-5258, *Lactococcus lactis* subsp. *Lactis* NCC 2471 having the accession number NCIMB 8586 and *Lactococcus lactis* subsp. *Lactis* NCC 2205 having the accession number CNCM I-5257, and

wherein the warm flavouring agent is selected among the group consisting of chocolate flavouring agent, vanilla flavouring agent, coffee flavouring agent, biscuit flavouring agent, cinnamon flavouring agent, caramel flavouring agent, praline flavouring agent, nougat flavouring agent, peanut flavouring agent, hazelnut flavouring agent, pistachio flavouring agent, almond flavouring agent, walnut flavouring agent, cashew nut flavouring agent and mixtures thereof.

6. A fermented dairy product according to claim 5 wherein the mesophilic strain having low post-acidifying properties has an enzymatic activity that leads to less than 0.22 pH units drop in a fermented dairy product containing between 2% and 4% of protein over 28 days of storage at 8°C after the twenty-four hours following the end of the fermentation .

7. A fermented dairy product according to any one of claims 5 to 6, having a fat content up to 5% based on the total weight of the fermented dairy product.

8. A fermented dairy product according to any one of claims 5 to 7, having a Dornic acidity ranging from 60°D to 80 °D.

9. A fermented dairy product according to any one of claim 5 to 8, consisting of a whipped fermented dairy product having an overrun ranging from 30% to 200%.

10. A multilayer food product comprising at least one layer of fermented dairy product according to any one of claim 5 to 9, or obtainable or obtained by a process according to any one of claims 1 to 4.

11. A mesophilic strain with low post-acidifying properties selected from the group consisting of *Lactococcus lactis* subsp. *lactis diacetylactis* NCC 2216 having the accession number CNCM I-5134, *Lactococcus lactis* subsp. *lactis diacetylactis* NCC 2272 having the accession number CNCM I-5258 and *Lactococcus lactis* subsp. *Lactis* NCC 2205 having the accession number CNCM I-5257.

**12.** A mesophilic strain with low post-acidifying properties according to claim 11, which has an enzymatic activity that leads to less than 0.22 pH units drop in a fermented dairy product containing between 2% and 4% of protein over 28 days of storage at 8°C after the twenty-four hours following the end of the fermentation.

**13.** A mesophilic strain with low post acidifying properties according to claims 11 or 12, which is suitable for use in a food product comprising a warm flavouring agent, wherein the warm flavouring agent is selected among the group consisting of chocolate flavouring agent, vanilla flavouring agent, coffee flavouring agent, biscuit flavouring agent, cinnamon flavouring agent, caramel flavouring agent, praline flavouring agent, nougat flavouring agent, peanut flavouring agent, hazelnut flavouring agent, pistachio flavouring agent, almond flavouring agent, walnut flavouring agent, cashew nut flavouring agent and mixtures thereof.

**Patentansprüche**

**1.** Verfahren zum Herstellen eines fermentierten Milchprodukts, das mindestens einen warmen Geschmacksstoff, einen pH-Wert zwischen 4,5 und 5,5 und einen Proteingehalt zwischen 2 % und 4 %, bezogen auf das Gesamtgewicht des fermentierten Milchprodukts, aufweist, umfassend die Schritte:

a) Zubereiten einer Milchzusammensetzung durch Mischen eines Ei-Milch-Pulvers mit einer Milchbasis

b) Homogenisieren der Milchzusammensetzung durch Hochdruckhomogenisierung bei einem Druck im Bereich von 250 bar bis 800 bar und einer Temperatur im Bereich von 60 °C bis 70 °C, um eine homogenisierte Milchzusammensetzung zu erhalten, Pasteurisieren der homogenisierten Milchzusammensetzung bei einer Temperatur im Bereich von 85 °C bis 95 °C für eine Zeit im Bereich von 3 Minuten bis 8 Minuten, um eine pasteurisierte Milchzusammensetzung zu erhalten,

c) Abkühlen der pasteurisierten Milchzusammensetzung auf eine Temperatur zwischen 32 °C und 35 °C und anschließendes Beimpfen der pasteurisierten Milchzusammensetzung mit einem mesophilen Stamm, um eine beimpfte Milchzusammensetzung zu erhalten, wobei der mesophile Stamm ein mesophiler Stamm ist, der geringe nachsäuernde Eigenschaften aufweist, und wobei der mesophile Stamm, der geringe nachsäuernde Eigenschaften aufweist, von der Gattung *Lactococcus* ist *und* aus der Gruppe ausgewählt ist, bestehend aus *Lactococcus lactis* subsp. *lactis diacetylactis* NCC 2216 mit der Zugangsnummer CNCM 1-5134, *Lactococcus lactis subsp. lactis diacetylactis* NCC 2272 mit der Zugangsnummer CNCM 1-5258, *Lactococcus lactis* subsp. *Lactis* NCC 2471 mit der Zugangsnummer NCIMB 8586 und *Lactococcus lactis* subsp. *Lactis* NCC 2205 mit der Zugangsnummer CNCM 1-5257,

d) Fermentieren der beimpften Milchzusammensetzung bei einer Temperatur zwischen 32 °C und 35 °C, bis ein pH-Wert im Bereich von 4,5 bis 5,5 erreicht ist, um ein fermentiertes Milchprodukt zu erhalten,

e) Mischen mindestens eines warmen Aromastoffs während Schritt a) und/oder nach Schritt d), wobei der warme Aromastoff aus der Gruppe ausgewählt ist, bestehend aus Schokoladenaromastoff, Vanillearomastoff, Kaffeearomastoff, Keksaromastoff, Zimtaromastoff, Karamellaromastoff, Pralinenaromastoff, Nougataromastoff, Erdnussaromastoff, Haselnussaromastoff, Pistazienaromastoff, Mandelaromastoff, Walnussaromastoff, Cashewnussaromastoff und Mischungen davon,

f) optional Abkühlen des fermentierten Milchprodukts, das den warmen Geschmacksstoff enthält, auf eine Temperatur von 8 °C.

**2.** Verfahren zum Herstellen eines fermentierten Milchprodukts nach Anspruch 1, wobei das Ei-Milch-Pulver hergestellt wird durch:

a') Mischen, unter Rühren, einer konzentrierten Milchsäurebasis, die reich an Milchproteinen ist, mit flüssigem Ei, um eine flüssige Ei-Milch-Mischung zu erhalten

b') Anpassen der flüssigen Ei-Milch-Mischung, falls erforderlich, auf einen Wert im Bereich von 6,5 bis 7,5

c') Pasteurisieren der flüssigen Ei-Milch-Mischung bei einer Temperatur im Bereich von 64 °C bis 75 °C für eine Zeit im Bereich von 5 Sekunden bis 150 Sekunden

d') Trocknen der pasteurisierten Mischung, um ein Ei-Milch-Pulver zu erhalten

**3.** Verfahren zum Herstellen eines fermentierten Milchprodukts nach Anspruch 2, wobei während Schritt c') die flüssige Ei-Milch-Mischung bei einer Temperatur im Bereich von 70 °C bis 75 °C für eine Zeit im Bereich von 10 bis 100 Sekunden pasteurisiert wird.

**4.** Verfahren zum Herstellen eines fermentierten Milchprodukts nach einem der Ansprüche 1 bis 3, wobei der mesophile Stamm, der geringe nachsäuernde Eigenschaften aufweist, eine enzymatische Aktivität aufweist, die bei einem fermentierten Milchprodukt, das zwischen 2 % und 4 % Protein enthält, über 28 Tage Lagerung bei 8 °C nach den vierundzwanzig Stunden nach dem Ende der Fermentation zu einem Abfall des pH-Werts von weniger als 0,22 Einheiten führt.

5. Fermentiertes Milchprodukt, das einen pH-Wert zwischen 4,5 und 5,5 und einen Proteingehalt im Bereich von 2 % bis 4 %, basierend auf dem Gesamtgewicht des fermentierten Milchprodukts, aufweist, umfassend einen mesophilen Stamm und mindestens einen warmen Geschmacksstoff, der durch das Verfahren nach einem der Ansprüche 1 bis 4 erhaltbar ist, wobei der mesophile Stamm ein mesophiler Stamm ist, der geringe nachsäuernde Eigenschaften aufweist,

wobei der mesophile Stamm aus der Gattung *Lactococcus* und aus der Gruppe ausgewählt ist, bestehend aus *Lactococcus lactis* subsp. *lactis diacetylactis* NCC 2216 mit der Zugangsnummer CNCM 1-5134, *Lactococcus lactis* subsp. *lactis diacetylactis* NCC 2272 mit der Zugangsnummer CNCM 1-5258, *Lactococcus lactis* subsp. *Lactis* NCC 2471 mit der Zugangsnummer NCIMB 8586 und *Lactococcus lactis* subsp. *Lactis* NCC 2205 mit der Zugangsnummer CNCM I-5257, und
wobei der warme Aromastoff aus der Gruppe ausgewählt ist, bestehend aus Schokoladenaromastoff, Vanillearomastoff, Kaffeearomastoff, Keksaromastoff, Zimtaromastoff, Karamellaromastoff, Pralinenaromastoff, Nougat Aromastoff, Erdnussaromastoff, Haselnussaromastoff, Pistazienaromastoff, Mandelaromastoff, Walnussaromastoff, Cashewnussaromastoff und Mischungen davon.

6. Fermentiertes Milchprodukt nach Anspruch 5, wobei der mesophile Stamm, der geringe nachsäuernde Eigenschaften aufweist, eine enzymatische Aktivität aufweist, die bei einem fermentierten Milchprodukt, das zwischen 2 % und 4 % Protein enthält, über 28 Tage Lagerung bei 8 °C nach den vierundzwanzig Stunden nach dem Ende der Fermentation zu einem Abfall des pH-Werts von weniger als 0,22 pH-Einheiten führt.

7. Fermentiertes Milchprodukt nach einem der Ansprüche 5 bis 6, das einen Fettgehalt von bis zu 5 %, bezogen auf das Gesamtgewicht des fermentierten Milchprodukts, aufweist.

8. Fermentiertes Milchprodukt nach einem der Ansprüche 5 bis 7, das einen Grad Dornic im Bereich von 60°D bis 80°D aufweist.

9. Fermentiertes Milchprodukt nach einem der Ansprüche 5 bis 8, bestehend aus einem geschlagenen fermentierten Milchprodukt, das einen Aufschlag im Bereich von 30 % bis 200 % aufweist.

10. Mehrschichtiges Nahrungsmittelprodukt, das mindestens eine Schicht aus fermentiertem Milchprodukt nach einem der Ansprüche 5 bis 9 umfasst oder durch ein Verfahren nach einem der Ansprüche 1 bis 4 erhaltbar ist oder erhalten wird.

11. Mesophiler Stamm, der geringe nachsäuernde Eigenschaften aufweist, der aus der Gruppe ausgewählt ist, bestehend aus *Lactococcus lactis* subsp. *lactis diacetylactis* NCC 2216 mit der Zugangsnummer CNCM 1-5134, *Lactococcus lactis* subsp. *lactis diacetylactis* NCC 2272 mit der Zugangsnummer CNCM 1-5258 und *Lactococcus lactis* subsp. *Lactis* NCC 2205 mit der Zugangsnummer CNCM I-5257.

12. Mesophiler Stamm, der geringe nachsäuernde Eigenschaften aufweist, nach Anspruch 11, der eine enzymatische Aktivität aufweist, die bei einem fermentierten Milchprodukt, das zwischen 2 % und 4 % Protein enthält, über 28 Tage Lagerung bei 8 °C nach den vierundzwanzig Stunden nach dem Ende der Fermentation zu einem Abfall des pH-Werts von weniger als 0,22 Einheiten führt.

13. Mesophiler Stamm, der geringe nachsäuernde Eigenschaften aufweist, nach Anspruch 11 oder 12, der zur Verwendung in einem Nahrungsmittelprodukt geeignet ist, das einen warmen Aromastoff umfasst, wobei der warme Aromastoff aus der Gruppe ausgewählt ist, bestehend aus Schokoladenaromastoff, Vanillearomastoff, Kaffeearomastoff, Keksaromastoff, Zimtaromastoff, Karamellaromastoff, Pralinenaromastoff, Nougataromastoff, Erdnussaromastoff, Haselnussaromastoff, Pistazienaromastoff, Mandelaromastoff, Walnussaromastoff, Cashewnussaromastoff und Mischungen davon.

**Revendications**

1. Procédé de préparation d'un produit laitier fermenté ayant au moins un agent aromatisant chaud, un pH compris entre 4,5 et 5,5 et une teneur en protéines comprise entre 2 % et 4 % sur la base du poids total du produit laitier fermenté, comprenant les étapes consistant à :

   a) préparer une composition laitière en mélangeant une poudre d'œufs et de lait avec une base laitière
   b) homogénéiser la composition laitière par homogénéisation à haute pression à une pression allant de 250 bars à 800 bars et à une température allant de 60 °C à 70 °C pour obtenir une composition laitière homogénéisée, pasteuriser la composition laitière homogénéisée à une température allant de 85 °C à 95 °C pendant une durée allant de 3 minutes à 8 minutes pour obtenir une composition laitière pasteurisée,
   c) refroidir la composition laitière pasteurisée à une température comprise entre 32 °C et 35 °C, puis inoculer la composition laitière pasteurisée avec une souche mésophile pour obtenir une composition laitière inoculée, dans lequel la souche mésophile est une souche mésophile ayant de faibles propriétés post-acidifiantes, et dans lequel la souche mésophile ayant de faibles propriétés post-acidifiantes appartient au genre *Lactococcus et* est choisie dans le groupe constitué de *Lactococcus lactis* subsp. *lactis diacetylactis* NCC 2216 ayant le numéro d'accession CNCM I-5134, *Lactococcus lactis subsp. lactis diacetylactis* NCC2272 ayant le numéro d'accession CNCM I-5258, *Lactococcus lactis* subsp. *Lactis* NCC 2471 ayant le numéro d'accession NCIMB 8586 et *Lactococcus lactis* subsp. *Lactis* NCC 2205 ayant le numéro d'accession CNCM I-5257,
   d) fermenter la composition laitière inoculée à une température comprise entre 32 °C et 35 °C jusqu'à l'obtention d'un pH allant de 4,5 à 5,5, afin d'obtenir un produit laitier fermenté,
   e) mélanger au moins un agent aromatisant chaud pendant l'étape a) et/ou après l'étape d), dans lequel l'agent aromatisant chaud est choisi dans le groupe constitué d'agent aromatisant au chocolat, agent aromatisant à la vanille, agent aromatisant au café, agent aromatisant aux biscuits, agent aromatisant à la cannelle, agent aromatisant au caramel, agent aromatisant à la praline, agent aromatisant au nougat, agent aromatisant aux cacahuètes, agent aromatisant à la noisette, agent aromatisant à la pistache, agent aromatisant à l'amande, agent aromatisant aux noix, agent aromatisant aux noix de cajou et des mélanges de ceux-ci,
   f) éventuellement, refroidir le produit laitier fermenté contenant ledit agent aromatisant chaud à une température de 8 °C.

2. Procédé de préparation d'un produit laitier fermenté selon la revendication 1, dans lequel la poudre d'œuf et de lait est préparée par :

   a') le mélange, sous agitation, d'une base lactique concentrée riche en protéines laitières avec de l'œuf liquide pour obtenir un mélange liquide œuf-lait
   b') l'ajustement du mélange liquide œuf-lait, le cas échéant, à une valeur allant de 6,5 à 7,5
   c') la pasteurisation du mélange œuf-lait liquide à une température allant de 64 °C à 75 °C pendant une durée allant de 5 secondes à 150 secondes
   d') le séchage du mélange pasteurisé pour obtenir une poudre d'œuf et de lait.

3. Procédé de préparation d'un produit laitier fermenté selon la revendication 2, dans lequel, au cours de l'étape c'), le mélange liquide œuf-lait est pasteurisé à une température allant de 70 °C à 75 °C pendant une durée allant de 10 à 100 secondes.

4. Procédé de préparation d'un produit laitier fermenté selon l'une quelconque des revendications 1 à 3, dans lequel la souche mésophile à faibles propriétés post-acidifiantes a une activité enzymatique qui conduit à une baisse inférieure à 0,22 unité de pH dans un produit laitier fermenté contenant entre 2 % et 4 % de protéines sur 28 jours de stockage à 8 °C après les vingt-quatre heures suivant la fin de la fermentation.

5. Produit laitier fermenté ayant un pH compris entre 4,5 et 5,5 et une teneur en protéines allant de 2 % à 4 % sur la base du poids total du produit laitier fermenté comprenant une souche mésophile et au moins un agent aromatisant chaud, pouvant être obtenu par le procédé de l'une quelconque des revendications 1 à 4, dans lequel la souche mésophile est une souche mésophile ayant de faibles propriétés post-acidifiantes,

   dans lequel la souche mésophile appartient au genre *Lactococcus* et est choisie dans le groupe constitué de *Lactococcus lactis* subsp. *lactis diacetylactis* NCC 2216 ayant le numéro d'accession CNCM I-5134, *Lactococcus lactis* subsp. *lactis diacetylactis* NCC 2272 ayant le numéro d'accession CNCM I-5258, *Lactococcus lactis* subsp. *Lactis* NCC 2471 ayant le numéro d'accession NCIMB 8586 et *Lactococcus lactis* subsp. *Lactis* NCC 2205 ayant le numéro d'accession CNCM I-5257, et

dans lequel l'agent aromatisant chaud est choisi dans le groupe constitué d'agent aromatisant au chocolat, agent aromatisant à la vanille, agent aromatisant au café, agent aromatisant aux biscuits, agent aromatisant à la cannelle, agent aromatisant au caramel, agent aromatisant à la praline, agent aromatisant au nougat, agent aromatisant aux cacahuètes, agent aromatisant à la noisette, agent aromatisant à la pistache, agent aromatisant aux amandes, agent aromatisant aux noix, agent aromatisant aux noix de cajou et des mélanges de ceux-ci.

6. Produit laitier fermenté selon la revendication 5, dans lequel la souche mésophile ayant de faibles propriétés post-acidifiantes a une activité enzymatique qui entraîne une baisse de moins de 0,22 unité de pH dans un produit laitier fermenté contenant entre 2 % et 4 % de protéines sur 28 jours de stockage à 8 °C après les vingt-quatre heures suivant la fin de la fermentation.

7. Produit laitier fermenté selon l'une quelconque des revendications 5 à 6, ayant une teneur en matières grasses inférieure ou égale à 5 % sur la base du poids total du produit laitier fermenté.

8. Produit laitier fermenté selon l'une quelconque des revendications 5 à 7, ayant une acidité Dornic allant de 60 °D à 80 °D.

9. Produit laitier fermenté selon l'une quelconque des revendications 5 à 8, consistant en un produit laitier fermenté fouetté ayant un taux de foisonnement allant de 30 % à 200 %.

10. Produit alimentaire multicouche comprenant au moins une couche de produit laitier fermenté selon l'une quelconque des revendications 5 à 9, ou pouvant être obtenu ou obtenu par un procédé selon l'une quelconque des revendications 1 à 4.

11. Souche mésophile à faibles propriétés post-acidifiantes choisie dans le groupe constitué de *Lactococcus lactis* subsp. *lactis diacetylactis* NCC 2216 ayant le numéro d'accession CNCM I-5134, *Lactococcus lactis* subsp. *lactis diacetylactis* NCC 2272 ayant le numéro d'accession CNCM I-5258 et *Lactococcus lactis* subsp. *Lactis* NCC 2205 ayant le numéro d'accession CNCM I-5257.

12. Souche mésophile à faibles propriétés post-acidifiantes selon la revendication 11, dont une activité enzymatique conduit à une baisse inférieure à 0,22 unité de pH d'un produit laitier fermenté contenant entre 2 % et 4 % de protéines sur 28 jours de stockage à 8 °C après les vingt-quatre heures suivant la fin de la fermentation.

13. Souche mésophile à faibles propriétés post-acidifiantes selon les revendications 11 ou 12, qui convient pour une utilisation dans un produit alimentaire comprenant un agent aromatisant chaud, dans lequel l'agent aromatisant chaud est choisi dans le groupe constitué d'agent aromatisant au chocolat, agent aromatisant à la vanille, agent aromatisant au café, agent aromatisant aux biscuits, agent aromatisant à la cannelle, agent aromatisant au caramel, agent aromatisant à la praline, agent aromatisant au nougat, agent aromatisant aux cacahuètes, agent aromatisant à la noisette, agent aromatisant à la pistache, agent aromatisant aux amandes, agent aromatisant aux noix, agent aromatisant aux noix de cajou et des mélanges de ceux-ci.

## REFERENCES CITED IN THE DESCRIPTION

**Patent documents cited in the description**

- WO 9812931 A
- WO 0019831 A
- WO 2004068958 A1
- US 20060068075 A1
- WO 2012136833 A1

- WO 200207541 A1
- CN 102952758 A
- WO 2016172570 A
- EP 2775853 B1

**Non-patent literature cited in the description**

- **AHMED et al.** Influence of temperature on growth pattern of Lactococcus lactis, Streptococcus cremoris and Lactobacillus acidophilus isolated from camel milk. *Biotechnology*, 2006, vol. 5 (4), 481-488
- **TANIGAWA et al.** Identification and typing of Lactococcus lactis by Matrix-Assisted Laser Desorption Ionization-Time of Flight Mass Spectrometry. *Applied and environmental microbiology*, June 2010, vol. 76 (12), 4055-4062

- **ZACHAROF et al.** Partially chemically defined liquid medium development for intensive propagation of industrial fermentation lactobacilli strains. *Annals of Microbiology*, 2013, vol. 63 (4), 1235-1245
- **ZACHAROF et al.** Separation of lactobacilli bacteriocins from fermented broths using membranes. *Process Biochemistry*, August 2013, vol. 48 (8), 1252-1261
- **OLASUPO et al.** Occurrence of nisin Z production in Lactococcus lactis BFE 1500 isolated from wara, a traditional Nigerian cheese product. *Int J Food Microbiol.*, December 1999, vol. 53 (2-3), 141-52